# EUROPEAN PATENT APPLICATION

(11) **EP 4 108 771 A1**
(43) Date of publication of application: **28.12.2022**
(21) Application number: 21181596.4
(22) Date of filing: 24.06.2021
(51) Int. Cl.: C12N 15/113, A61K 31/7125, C12N 15/115

(54) **G- QUADRUPLEX- CONTAINING OLIGONUCLEOTIDES FOR PREVENTIVE AND THERAPEUTIC TREATMENT**

(71) Applicant: Johann-Wolfgang-Goethe-Universität Frankfurt am Main, 60323 Frankfurt am Main (DE)
(72) Inventor: KIPPENBERGER, Stefan, 60316 Frankfurt (DE); STEINHORST, Katja, 61267 Neu Anspach (DE); CINATL, Jindrich, 63069 Offenbach (DE); BOJKKOVA, Denisa, 60528 Frankfurt (DE)
(74) Representative: Kehl, Ascherl, Liebhoff & Ettmayr Patentanwälte Partnerschaft mbB

(57) **Abstract**

The present invention refers to an oligonucleotide molecule having from 10 to 50 nucleotides comprising at least one G-quartet forming motif comprising 10 to 20 nucleotide residues, wherein at least 60% of said G-quartet forming motive forming residues are guanosine or deoxyguanosine residues, and wherein the molecule inhibits viral or bacterial replication and/or exerts antiinflammatory effects in mammalian cells.

SEQUENCE LISTING

<110>  Johann Wolfgang Goethe-Universität

<120>  G- quadruplex- containing oligonucleotides for preventive and
therapeutic treatment

<130>  6029-1-EP

<140>  EP21181596.4
<141>  2021-06-24

<160>  18

<170>  PatentIn version 3.5

<210>  SEQ. ID No. 1
<211>  20
<212>  DNA
<213>  Artificial Sequence
<223>  all guanosines are linked by phosphorothioate bonds
<400>
gggggggggg gggggggggg                                                  20

<210>  SEQ. ID No. 2
<211>  14
<212>  DNA
<213>  Artificial Sequence
<223>  all guanosines are linked by phosphorothioate bonds
<400>
gggggggggg gggg                                                        14

<210>  SEQ. ID No. 3
<211>  20
<212>  DNA
<213>  Artificial Sequence
<223>  n = guanosines linked by phosphorothioate bond

<400>  3
ngggtcaagc ttgannnnng                                                  20

<210>  SEQ. ID No. 4
<211>  26
<212>  DNA
<213>  Artificial Sequence
<223>  all nucleosides are linked by phosphorothioate bonds
<400>
ggtggtggtg gttgtggtgg tggtgg                                           26

<210>  SEQ. ID No. 5, CpG-1-PTO
<211>  20
<212>  DNA
<213>  Artificial Sequence
<223>  all nucleosides are linked by phosphorothioate bonds
<400>  5
tccatgacgt tcctgacgtt                                                  20

<210>  SEQ. ID No. 6, n-CpG-6-PTO
<211>  20

<212>  DNA
<213>  Artificial Sequence
<223>  all guanosines are linked by phosphorothioate bonds
<400>  6
gggggggggg gggggggggg                                                    20


<210>  SEQ. ID No. 7, n-CpG-3A-PTO
<211>  18
<212>  DNA
<213>  Artificial Sequence
<223>  all thymidines are linked by phosphorothioate bonds
<400>  7
tttttttttt tttttttt                                                      18


<210>  SEQ. ID No. 8, n-CpG-5-PTO
<211>  20
<212>  DNA
<213>  Artificial Sequence
<223>  all cytidines are linked by phosphorothioate bonds
<400>  8
cccccccccc cccccccccc                                                    20


<210>  SEQ. ID No. 9, Scramb-CpG-1-PTO
<211>  20
<212>  DNA
<213>  Artificial Sequence
<223>  all nucleosides are linked by phosphorothioate bonds
<400>  9
ctctaggact ctctggactt                                                    20


<210>  SEQ. ID No. 10, G3139 Genasense
<211>  18
<212>  DNA
<213>  Artificial Sequence
<223>  all nucleosides are linked by phosphorothioate bonds
<400>  10
tctcccagcg tgcgccat                                                      18


<210>  SEQ. ID No. 11, CpG-2118
<211>  20
<212>  DNA
<213>  Artificial Sequence
<223>  n = guanosines linked by phosphorothioate bond
<400>  11
nnggtcaagc ttgannnnng                                                    20


<210>  SEQ. ID No. 12, nCpG-6C-PTO
<211>  14
<212>  DNA
<213>  Artificial Sequence
<223>  all guanosines are linked by phosphorothioate bonds
<400>  12
gggggggggg gggg                                                          14


<210>  SEQ. ID No. 13, nCpG-6G-PTO
<211>  6

<212>  DNA
<213>  Artificial Sequence
<223>  all guanosines are linked by phosphorothioate bonds
<400>  13
gggggg                                                                 6


<210>  SEQ. ID No. 14, nCpG-6-PTO
<211>  20
<212>  DNA
<213>  Artificial Sequence
<223>  all guanosines are linked by phosphorothioate bonds
<400>  14
gggggggggg gggggggggg                                                 20


<210>  SEQ. ID No. 15, AS1411-PDE
<211>  26
<212>  DNA
<213>  Artificial Sequence
<400>  15
ggtggtggtg gttgtggtgg tggtgg                                          26


<210>  SEQ. ID No. 16, AS1411-PTO
<211>  26
<212>  DNA
<213>  Artificial Sequence
<223>  all nucleosides are linked by phosphorothioate bonds
<400>  17
ggtggtggtg gttgtggtgg tggtgg                                          26


<210>  SEQ. ID No. 17, Quench2
<211>  31
<212>  DNA
<213>  Artificial Sequence
<223>  all nucleosides are linked by phosphorothioate bonds
<400>  17
cgcagtcttc tcctggtgct cgaacagtga c                                    31


<210>  SEQ. ID No. 18, Flu2
<211>  18
<212>  DNA
<213>  Artificial Sequence
<223>  all nucleosides are linked by phosphorothioate bonds
<400>  18
gtcactgttc gagcacca                                                   18

SEQUENCE LISTING

<110>  Johann Wolfgang Goethe-Universität

<120>  G- quadruplex- containing oligonucleotides for preventive and
therapeutic treatment

<130>  6029-1

<140>  EP21181596.4
<141>  2021-06-24

<160>  18

<170>  PatentIn version 3.5

<210>  1
<211>  20
<212>  DNA
<213>  Artificial Sequence
<220>
<221>  n-CpG-6-PTO
<222>  (1)...(20)
<223>  all guanosines are linked by phosphorothioate bonds
<400>  1
gggggggggg gggggggggg                                                    20

<210>  2
<211>  14
<212>  DNA
<213>  Artificial Sequence
<220>
<221>  n-CpG-6C-PTO
<222>  (1)...(14)
<223>  all guanosines are linked by phosphorothioate bonds
<400>  2
gggggggggg gggg                                                          14

<210>  3
<211>  20
<212>  DNA
<213>  Artificial Sequence
<220>
<221>  CpG-2118 (KonA)
<222>  (1),(2),(15)...(19)
<223>  n indicates guanosines linked by phosphorothioate bonds
<400>  3
nnggtcaagc ttgannnnng                                                    20

<210>  4
<211>  26
<212>  DNA
<213>  Artificial Sequence
<220>
<221>  AS1411-PTO
<222>  (1)... (26)
<223>  all nucleosides are linked by phosphorothioate bonds
<400>  4
ggtggtggtg gttgtggtgg tggtgg                                             26

<210>  5
<211>  20
<212>  DNA
<213>  Artificial Sequence
<220>
<221>  CpG-1-PTO
<222>  (1)... (20)
<223>  all nucleosides are linked by phosphorothioate bonds
<400>  5
tccatgacgt tcctgacgtt                                                    20

<210>  6
<211>  18
<212>  DNA
<213>  Artificial Sequence
<220>
<221>  n-CpG-3A-PTO
<222>  (1)...(18)
<223>  all thymidines are linked by phosphorothioate bonds
<400>  6
   tttttttttt tttttttt                                                        18

<210>  7
<211>  20
<212>  DNA
<213>  Artificial Sequence
<220>
<221>  n-CpG-5-PTO
<222>  (1)...(20)
<223>  all cytidines are linked by phosphorothioate bonds
<400>  7
cccccccccc cccccccccc                                                    20

<210>  8
<211>  20
<212>  DNA
<213>  Artificial Sequence
<220>
<221>  Scramb-CpG-1-PTO
<222>  (1)...(20)
<223>  all nucleosides are linked by phosphorothioate bonds
<400>  8
ctctaggact ctctggactt                                                    20

<210>  9
<211>  18
<212>  DNA
<213>  Artificial Sequence
<220>
<221>  G3139 Genasense
<222>  (1)...(18)
<223>  all nucleosides are linked by phosphorothioate bonds
<400>  9
tctcccagcg tgcgccat                                                      18

<210>  10
<211>  6

<212>  DNA
<213>  Artificial Sequence
<220>
<221>  nCpG-6G-PTO
<222>  (1)...(6)
<223>  all guanosines are linked by phosphorothioate bonds
<400>  110
gggggg                                                                 6

<210>  11
<211>  26
<212>  DNA
<213>  Artificial Sequence
<400>  11
ggtggtggtg gttgtggtgg tggtgg                                          26

<210>  12
<211>  31
<212>  DNA
<213>  Artificial Sequence
<220>
<221>  Quench2
<222>  (1)...(31)
<223>  all guanosines are linked by phosphorothioate bonds
<400>  12
cgcagtcttc tcctggtgct cgaacagtga c                                    31

<210>  13
<211>  18
<212>  DNA
<213>  Artificial Sequence
<220>
<221>  Flu2
<222>  (1)...(18)
<223>  all guanosines are linked by phosphorothioate bonds
<400>  13
gtcactgttc gagcacca                                                   18

<210>  14
<211>  13
<212>  DNA
<213>  Artificial Sequence
<220>
<221>  Capture Q2
<222>  (1)...(13)
<223>  all nucleosides are linked by phosphorothioate bonds
<400>  14
gctactgtgt gtg                                                        13

<210>  15
<211>  20
<212>  DNA
<213>  Artificial Sequence
<400>  15                                                                20
gggggggggg gggggggggg

## Description

### Invention

The invention relates to an oligonucleotide molecule according to the subject matter of claim 1. The invention also relates to a pharmaceutical composition according to the subject matter of claim 9, a kit comprising the oligonucleotide molecule of the invention according to the subject matter of claim 10 and to therapeutic uses of the inventive oligonucleotide molecule.

### Background

Infectious diseases, such as diseases caused by viruses or bacteria, impose significant health issues to patients and healthcare systems. For instance, seasonal respiratory viral diseases have been known for thousands of years, as annual epidemics of common cold and of influenza disease affect humans living in temperate regions regularly during the winter season. Newly emerging viral infections continue to pose a major threat to global public health. Recently, highly pathogenic avian influenza A (H5N1) viruses was as well as other avian influenza A virus subtypes (H7N9, H9N2, and H7N3) were found to be associated with human disease, raising the concern of pandemic conditions due to the potential spread of subtypes of influenza A virus circulating in domestic and wild birds and livestock to humans. Severe acute respiratory syndrome Corona viruses (SARS-CoV) are implicated in an atypical pneumoniae, which emerged first in 2002 and 2003 in Guandong province (China), resulting in 800 deaths worldwide in 2003. Bats have been identified as the natural reservoir of SARS-CoV-like viruses. In 2012, Middle East Respiratory Syndrome Corona viruses, belonging to the same group of Corona viruses were identified in Saudi Arabia. In late 2019, a pneumonia associated with SARS-CoV2 infection emerged in patients in the city of Wuhan (China), leading to the COVID-19 pandemic condition affecting millions of people and resulting in millions of deaths around the globe. SARS-CoV viruses belong to the family of betacoronaviruses and are positive single-stranded (ss) RNA viruses with a large RNA genome of approximately 30 Kb. Like other coronaviruses, the SARS-CoV2 genome contains 14 open reading frames (ORFs) encoding 27 proteins. The ORF1 and ORF2 at the 5'-terminal region of the genome encode 15 non-structural proteins important for virus replication. The 3'-terminal region of the genome encodes structural protein, in particular a spike protein (S), an envelope protein (E), a membrane protein (M), and a nucleocapsid protein (N) and also eight different accessory proteins.

There is a need for effective drugs or vaccines available for treating emerging viral diseases, in particular for treating coronavirus infections. Small molecule inhibitors may be easily identified from compound libraries. Their action, however, is confined to a small surface area of a given target such that single amino acid changes in the target may result in a significantly reduced efficacy of a given small molecule inhibitor. Oligonucleotide molecules which bind to specific target molecules ("aptamers") may be isolated using Systematic Evolution of Ligands by EXponential enrichment (SELEX) and may serve as affinity probes or molecular recognition elements for diagnostic or therapeutic purposes. These aptamers are synthetic single-stranded DNAs or RNAs, which bind to target molecules with high affinity in three-dimensional shapes and which have been applied widely in analytical, bioanalytical, imaging, diagnostic and therapeutic fields. The present invention addresses the need for effective drugs for treating diseases caused by viral or bacterial infections using oligonucleotide molecules.

### Summary of the invention

In a first aspect, the invention relates to an oligonucleotide molecule having from 10 to 50 nucleotides comprising at least one G-quartet forming motif comprising 10 to 20 nucleotide residues, wherein at least 60% of the residues are guanosine or deoxyguanosine residues, and wherein the molecule inhibits viral or bacterial replication and/or exerts anti-inflammatory effects in a mammalian cell.

According to the invention, the term "oligonucleotide molecule" is understood to refer to a short DNA or RNA molecule, also known as oligomer. Most commonly, DNA oligonucleotides are synthesized as single-stranded molecules by solid-phase chemical synthesis and are used for artificial gene synthesis, polymerase chain reaction (PCR), DNA sequencing, molecular cloning and as molecular probes. RNA oligonucleotides occur as small RNA molecules in vivo, where they are involved in the regulation of gene expression (e.g., microRNA), or are degradation intermediates derived from the breakdown of larger nucleic acid molecules. Oligonucleotides which bind to specific target molecules are called "aptamers", wherein aptamers may be oligonucleotide molecules but also peptide molecules. Within the context of the invention, the term "aptamer" refers to an oligonucleotide molecule; the terms "oligonucleotide molecule" and "aptamer" are used interchangeably and synonymously throughout this application.

In the context of the present invention, a "G-quartet forming motif" is a higher-order nucleic acid structure rich in guanine residues. G-quartets are formed by four G-bases which are associated via Hoogsteen H-bonding to form a square planar structure such that each G-base makes two H-bonds with its neighboring G-base. To form a G-quadruplex (G4), two or more G-quartets stack on top of each other, thus forming polymorphic structures. Therefore, a folded intramolecular G-quadruplex consists of two main elements: core and loops, wherein the core comprises one or more stacked G·G·G·G tetrad (or G-quartet) layers, while the loops are linker sequences connecting the strands of the G-tetrad core. Hence, G-quadruplex structures are highly polymorphic, depending on the relative orientations of strands and types of loops. Depending on the sequence of the G-rich oligonucleotides, stability of these G-quartets is related to several factors, including the presence of monovalent cations such as K+ and Na+, the concentration of G-rich oligonucleotides present, and the sequence of the G-rich oligonucleotides being used.

The oligonucleotide molecule of the invention is able to inhibit viral or bacterial replication, wherein replication of viral or bacterial genomes result in the production of multiple copies of the virus in infected cells or of the bacterium. It is preferred, that the oligonucleotide molecule of the invention targets a viral or a bacterial helicase. Helicases are enzymes which separate the strands of a duplex nucleic acid, usually using the hydrolysis of ATP to provide the necessary energy. In addition to the helicases that act on double-stranded DNA, some helicases unwind DNA-RNA or RNA-RNA duplexes. Concerning bacterial replication, it is also preferred that the oligonucleotide molecule of the invention may target the DNA polymerase III holoenzyme of bacteria, which is the primary enzyme complex involved in prokaryotic DNA replication. Since viral replication primarily depends on metabolic functions of the host cell, the oligonucleotide molecule of the invention advantageously targets a virus-specific step in the viral replication mechanism and leaves host cell functions intact. Different viral polymerases, i.e., RNA-dependent RNA polymerase, RNA-dependent DNA polymerase, DNA-dependent RNA polymerase, and DNA-dependent DNA polymerases, play a central role in viral replication and transcription of the viral genome and are generally active as a single protein capable of carrying out multiple functions related to viral replication. It is also preferred, when used in order to inhibit viral replication, the oligonucleotide molecule of the invention may target one or more viral polymerase.

The term "inflammation" refers to a complex biological response of one or more tissues to harmful stimuli, such as, e.g., viral or bacterial pathogens. The response serves to eliminate the initial cause of the cell injury in order to protect tissue cells. In mammals, an acute inflammatory reaction is an immediate, adaptive response with limited specificity which is generally considered beneficial. It can become detrimental if not regulated, however, such as seen in septic shock. The inflammatory pathway consists of a sequence of events involving inducers, sensors, mediators, and effectors. The "anti-inflammatory effect" of a molecule or a substance refers to properties reducing inflammation, for example, by a specific interaction of said molecule with one or more of said inducers, sensors, mediators, and effectors.

Surprisingly, the oligonucleotide molecule of the invention, wherein at least 80% of the residues are guanosine or deoxyguanosine residues, were found to exhibit strong antiviral or antibacterial activity, via inhibition of replication. Moreover, the molecule was also found to exert anti-inflammatory effects in mammalian cells. Advantageously, the oligonucleotide molecules of the invention are short molecules which may be synthesized relatively easily by chemical synthesis at low cost. Compared to antibodies, they are characterized by minimal immunogenicity and high stability. Furthermore, they are able to bind to a variety of targets comprising organic molecules, proteins, viruses, bacteria, whole cells and tissues.

In a further embodiment, the oligonucleotide molecule according to the invention may be an oligonucleotide molecule, wherein > 70% of the residues are guanosine or deoxyguanosine residues. Increasing the number of guanosine residues results in the oligonucleotide of the invention exhibiting significantly increased antiviral or antibacterial activity, when compared to scrambled or mixed sequences. It is particularly preferred, when all residues in the oligonucleotide molecule are guanosine residues.

In an alternative embodiment, the oligonucleotide molecule may be synthesized from deoxynucleotides. An individual nucleotide is composed of three subunit molecules: a nucleobase, a five-carbon sugar (ribose or deoxyribose), and a phosphate group consisting of one to three phosphates. The nucleobase and the sugar moiety together form a nucleoside. In DNA, the nucleobases used are guanine, adenine, cytosine and thymine; in RNA, uracil is used in place of thymine. In RNA, the sugar in the sugar-phosphate backbone is ribose, wherein DNA is a nucleic acid polymer characterized by the presence of deoxyribose instead. Advantageously, the oligonucleotide molecule is a DNA molecule with much lower susceptibility to hydrolysis.

In further advantageous embodiment of the inventive oligonucleotide molecule, at least one of the guanosine or deoxyguanosine residues may be chemically modified. The oligonucleotide molecule is either a DNA or an RNA molecule, which may be modified chemically at the backbone or on the 2' sugar position to achieve different effects such as higher binding affinity and/or specificity, lower susceptibility to nuclease degradation, enhanced in vivo stability, longer in vivo half-life, decreased susceptibility to excretion via renal filtration. Common chemical modifications involve modifications of the terminals of nucleic acids, such as 3' end capping with inverted thymidine and PEGylation in order to improve resistance against nucleases (which first bind at the respective terminals) and renal clearance, respectively. Further modifications involve the phosphodiester linkage, the sugar ring (substituting, e.g., the 2'O position of the ribofuranose ring with fluoro- (-F), amino (-NH2), azido (-N3) or methoxy/OMe (-OCH3) groups) and the nucleobases (such as, e.g., the purine modifications 2,6-diaminopurine, 3-deaza-adenine, 7-deaza-guanine and 8-azido-adenine, or the pyrimidine modifications 2-thio-thymidine, 5-carboxamide-uracil, 5-methyl-cytosine and 5-ethynyl-uracil).

In a particularly preferred embodiment of the oligonucleotide molecule, the chemical modification may be a phosphate backbone modification. A modification of the phosphate backbone by definition affects the phosphodiester linkage, wherein the phosphate group is altered by atomic substitutions, resulting in neutral, anionic or cationic modifications. For instance, replacing one or two of the oxygen atoms by one or two sulfur atoms, respectively, yields phosphorothioate or phosphorodithioate groups. Replacing one oxygen atom of the phosphate group with an uncharged methyl group results in a methyl phosphate backbone. A cationic modification involves the replacement of one oxygen atom with a positively charged group such as guanidinopropyl phosphoramidate. Preferably, the phosphodiester linkage of the oligonucleotide molecule is replaced with the methylphosphonate or the phosphorothioate analog, such that a backbone O atom is replaced with either a methyl group or one or more backbone O atom is replaced with one or more sulfur atoms. Advantageously, the modification results in improved resistance to extracellular or intracellular nucleases, higher thermal stability, improved target binding affinity and/or improved delivery via the plasma membrane to the interior of the cell.

It is particularly preferred if the thiophosphoryl substitutions are selected from phosphorothioate or phosphorodithioate, wherein thiophosphoryl substitutions replace at least 35 % of the phosphodiester linkages in the sugar-phosphate backbone of the oligonucleotide molecule. Substitution of all phosphodiester linkages in the oligonucleotide by thiophosphoryl groups leads to significantly enhanced resistance to nuclease. In order to obtain oligonucleotide molecules with enhanced target binding specificity, thiophosphoryl substitutions of the phosphodiester linkages may be titrated within the range of 35% to complete replacement. Furthermore, by a partial substitution and thus preservation of some phosphodiester linkages within the molecule, an enhanced toxicity, which sometimes is found to be associated with complete substitution, may be avoided. When in an oligonucleotide molecule, wherein thiophosphoryl substitutions replace at least 35 % of the phosphodiester linkages in the sugar-phosphate backbone, the number of guanosine residues is increased, the oligonucleotide molecule of the invention exhibits significantly increased antiviral or antibacterial activity, when compared to scrambled or mixed sequences. It is particularly preferred, when all residues in said PTO oligonucleotide molecule are guanosine residues.

In a further embodiment, the oligonucleotide molecule may comprise at least four consecutive triplets of guanosine or deoxyguanosine residues. Advantageously, oligonucleotide molecules comprising at least four consecutive triplets of guanosine residues were found to exhibit a stronger anti-viral effect compared to shorter oligonucleotide molecules, suggesting that the efficacy to inhibit viral replication depends on the length of the molecule.

The oligonucleotide of the invention may comprise advantageously the sequence set forth in SEQ ID NOs. 1 to 4, wherein SEQ ID NO. 1: 5'- GGG GGG GGG GGG GGG GGG GG -3', SEQ ID NO. 2: 5'- GGG GGG GGG GGG GG -3', SEQ ID NO. 3; 5' GGg gtc aag ctt gaG GGG Gg and SEQ ID NO4: GGT GGT GGT GGT TGT GGT GGT GGT GG. Capital letters symbolize phosphorothioate bonds (PTO), lowercase letters symbolize classic phosphodiester bonds. Shorter sequences often are associated with a slightly reduced inhibition of replication. Oligonucleotides comprising those sequences spontaneously form G-quartets and G quadruplexes (G4s).

In a second aspect, the invention relates to a pharmaceutical composition comprising at least one oligonucleotide molecule combined with at least one of a pharmaceutically acceptable excipient, carrier, adjuvant or combination thereof.

A pharmaceutically acceptable excipient according to the present invention includes any and all solvents, dispersion media, diluents, or other liquid vehicles, dispersion or suspension aids, surface active agents, isotonic agents, preservatives, solid binders and the like as suited to the particular dosage form desired. Remington's The Science and Practice of Pharmacy, 23rd Edition, A. Adejare, (Lippincott, Williams & Wilkins, Baltimore, Md., 2020) discloses various excipients used in formulating pharmaceutical compositions and known techniques for the preparation thereof. The pharmaceutically acceptable excipient may be at least 95%, 96%, 97%, 98%, 99%, or 100% pure. Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils.

In a third aspect, the invention relates to a kit comprising at least one oligonucleotide molecule as described before.

In a fourth aspect, the invention relates to an oligonucleotide molecule as described, a pharmaceutical composition or a kit comprising the oligonucleotide molecule for use in treatment of a disease caused by a viral or a bacterial infection and/or of inflammation associated therewith.

In a preferred embodiment, the oligonucleotide molecule may induce inhibition of viral replication in a mammalian cell.

In a further preferred embodiment, the viral infection may be caused by a virus selected from the group comprising HS-1 virus, HCN virus, Adenovirus, Zika virus, hepatitis B or C virus, West Nile virus, influenza virus, RSV virus, Paramyxo-Virus, HIV virus, and corona viruses, such as SARS-CoV-1, SARS-CoV-2 and MERS-CoV.

In an alternative embodiment, the disease may be a viral infection of the respiratory tract.

In another embodiment, the treatment of the viral or bacterial disease-associated inflammation may result from interference with the type I interferon (IFN) and/or the type II IFN pathway or from suppression of interleukin mediated signaling. In the context of the present invention, type I interferons (IFNs) refer to polypeptides secreted by infected cells. The following functions are associated with type I IFNs: Induction of cell-intrinsic antimicrobial states in infected and neighbouring cells in order to limit the spread of infectious agents, particularly viral pathogens; modulation of innate immune responses in a balanced manner in order to promote antigen presentation and natural killer cell functions while restraining pro-inflammatory pathways and cytokine production; activation of the adaptive immune system, in order to promote the development of high-affinity antigen-specific T and B cell responses and immunological memory. Type I IFNs consist of a group of structurally similar cytokines and include 13-14 subtypes of IFN-α along with IFN-β, IFN-ε, IFN-κ, IFN-ω, IFN-δ, IFN-ζ, and IFN-τ. Type II IFN, known as IFN-γ, signals through a different receptor and has effects that are independent from type I IFN. IFN-γ signaling plays a key role in host defense by promoting macrophage activation, upregulating the expression of antigen processing and presentation molecules, driving the development and activation of Th1 cells, enhancing natural killer cell activity, regulating B cell functions, and inducing the production of chemokines that promote effector cell trafficking to sites of inflammation. Advantageously, the oligonucleotide molecule may be used for treatment at different stages of a viral disease, e.g., in a subacute state of a SARS-Cov2 infection ("long COVID").

In a further embodiment, in the oligonucleotide molecule used for treatment purposes, at least one of the guanosine or deoxyguanosine residues may be chemically modified, wherein the chemical modification is a phosphate backbone modification, and wherein the oligonucleotide comprises at least four consecutive triplets of guanosine or deoxyguanosine residues.

In an alternative embodiment, the molecule may be administered in a pharmaceutically acceptable route selected from the group consisting of orally, parenterally, enterally, via the ophthalmic or nasal route, or topically and combinations thereof. Topical applications comprise application as cream, foam, gel, lotion, ointment, paste, powder, shake lotion, solid, sponge, tape, tincture, topical solution, nail polish, transdermal patch, vapor.

It should be noted that, as used herein and in the claims, the singular forms "a", "an", and "the" include plural referents unless the context clearly dictates otherwise. For example, the term "an oligonucleotide molecule" refers to one or more oligonucleotide molecules, i.e., a single oligonucleotide molecule and multiple oligonucleotide molecules. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

Unless otherwise defined, all technical and scientific terms used herein have the same meaning as commonly understood by the skilled person which the description of the invention is directed at. Although methods and materials similar or equivalent to those described herein may be used in order to practice the present invention, suitable methods and materials are described below. In addition, the materials, methods, and examples described herein are illustrative only and are not intended to be limiting.

Other features and advantages of the invention will be apparent from and encompassed by the following detailed description and claims.

### Brief description of the drawings

Fig. 1 depicts a comparison of different nucleotide sequences for antiviral effect in SARS-CoV-2 infected Calu-3 human lung cancer cells. it is demonstrated that nCpG-6-PTO inhibits SARS-CoV-2 infections.
In Fig. 2 it is shown that nCpG-6-PTO prevents replication of SARS-CoV-2, but has no impact on virus entry into target cells.
   That the antiviral efficacy depends on the length of the molecule is demonstrated in Fig. 3, emphasizing that long molecules displayed stronger effect on virus infection than the shorter ones.
Fig. 4 demonstrates that the antiviral effect depends on the ODN-backbone by comparison of oligonucleotides of equal length with either a phosphodiester backbone (nCpG-6-PDE) or a phosphorothioate backbone (nCpG-6-PTO).
In Fig. 5, nCpG-6-PTO is compared to the efficacy of the well-known aptamer AS1411 used in anti-cancer therapy. Used in a virus inhibition assay, AS1411-PDE exhibited no efficacy against SARS-CoV-2, AS1411-PTO; however, was found as effective as CpG-6-PTO.
Fig. 6 shows that nCpG-6-PTO completely suppresses the cytopathogenic effect caused by HSV-1 at a concentration ranging from 0.5, 1, 2, 4µM.
In Fig. 7, the formation of quadruplex (G4) structures from nCpG-6-PTO is demonstrated by using BG4, a G4-specific antibody.
   By using a helicase assay, it is demonstrated that nCpG-6-PTO inhibits the SARS-CoV-2 helicase; results are shown in Fig. 8.
In Fig. 9, it is shown that G4-forming PTO-ODN (nCpG-6-PTO and AS1411-PTO) may inhibit IFNβ-mediated signaling molecules. Fig. 9A depicts a schematic pathway of type I interferon signaling (from Gonzalez-Cao *et al.*, 2018). By Western blot analysis (Fig. 9B), strong downregulation of tyrosine phosphorylation of the canonical signaling molecules p-Stat1 and pStat2 by nCpG-6-PTO and AS1411-PTO is shown.
Fig. 10 demonstrates that G4-forming PTO-ODN (nCpG-6-PTO and AS1411-PTO) inhibit IFNy-mediated signaling molecules. Fig. 10A depicts a schematic pathway of type II interferon signaling (from Gonzalez-Cao *et al.*, 2018). By Western blot analysis (Fig. 10B), strong downregulation of tyrosine phosphorylation of the canonical signaling molecules p-JAK2 and pStat2 by nCpG-6-PTO and AS1411-PTO is shown.
In Fig. 11, it is shown that G4-forming PTO-ODN (nCpG-6-PTO and AS1411-PTO) may inhibit interleukin 6 (IL-6) mediated-mediated STAT 3 phosphorylation. Fig. 11A depicts a schematic pathway of IL 6 signaling (from Jin *et al.*, 2017). By Western blot analysis (Fig. 9B), strong downregulation of tyrosine phosphorylation of Stat 3 by nCpG-6-PTO and AS1411-PTO is shown. Moreover, suppressive effects on p-Stat1 were also found.

### Detailed description of the invention:

### Example 1:

### G-rich PTO-ODN offer antiviral activity with nCpG-6-PTO being particularly potent

### Virus preparation

Virus preparation was performed as described (Bojkova *et al.*, 2020). Briefly, SARS-CoV-2 variants were isolated using the human colon carcinoma cell line Caco-2. SARS-CoV-2 stocks used in the experiments had undergone maximum three passages on Caco-2 cells and were stored at -80 °C. Virus titers were determined as TCID₅₀/ml in confluent cells in 96-well microtiter plates.

### Antiviral assay

Confluent layers of Calu-3 cells in 96-well plates were infected with SARS-CoV-2 at a MOI of 0.01. The term "MOI" denotes "multiplicity of infection" and refers to the ratio of agents (e.g., viruses, bacteria) to infection targets (e.g., cells). Virus was added together with the oligonucleotides at the same time and incubated in MEM supplemented with 1% FBS. The antiviral effects were assessed after 2 days by immunohistochemical detection of a virus-specific antigen using antibodies against SARS-CoV-2 S (1:1500, Sino Biological, Eschborn, Germany). The quantitative detection was performed using the Bioreader^{®} 7000 -F-Z-I micro (Biosys)

### Oligonucleotide molecules (ODN) used

1. CpG-1-PTO: 5'-TCC ATG ACG TTC CTG ACG TT-3'
2. n-CpG-6-PTO: 5'- GGG GGG GGG GGG GGG GGG GG -3'
3. n-CpG-3A-PTO: 5'- TTT TTT TTT TTT TTT TTT -3'
4. n-CpG-5-PTO: 5'- CCC CCC CCC CCC CCC CCC CC -3'
5. Scramb-CpG-1-PTO: 5'-CTC TAG GAC TCT CTG GAC TT-3'
6. G3139 Genasense (Oblimersen): 5'-TCTCCCAGCGTGCGCCAT-3'
7. CpG-2118(KonA): 5'-GGg gtc aag ctt gaG GGG Gg-3'

Capital letters symbolize phosphorothioate bonds (PTO), lowercase letters symbolize classic phosphodiester bonds (CpG-2118). As used herein, CpG oligodeoxynucleotides are short single-stranded synthetic DNA molecules that contain a cytosine triphosphate deoxynucleotide ("C") followed by a guanine triphosphate deoxynucleotide ("G"), wherein "p" refers to the phosphodiester link between consecutive nucleotides. The acronym "n-CpG" refers to oligonucleotides, which are non-CpG-ODN. Different concentrations were tested (0.25, 0.5, 1, 2 and 4 mM). Oblimersen (tradename: Genasense) is an antisense oligodeoxyribonucleotide being studied as a possible treatment for several types of cancer, including chronic lymphocytic leukemia, B-cell lymphoma, and breast cancer. CpG-2118(KonA); a guanine-rich molecule protected at the ends by PTO bonds, is a synthetic oligonucleotide serving as control for ODN 1585, a murine TLR9 ligand.

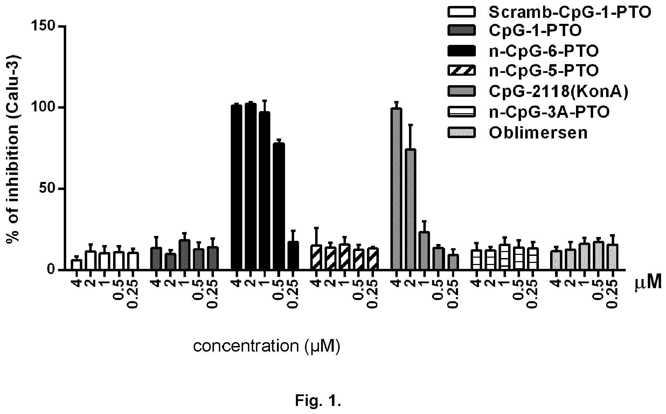

Results are depicted in Fig. 1, demonstrating that nCpG-6-PTO inhibits SARS-CoV-2 infections.

### Example 2:

### nCpG-6-PTO inhibits "replication", not "entry" of SARS-CoV-2.

### Materials & Methods

In order to discriminate if nCpG-6-PTO prevents virus adsorption and internalization into cells (entry) or virus replication (replication) the time-of-addition experiment was performed:
Time-of-addition experimental set up:
Entry: Different concentration of nCpG-6-PTO (0.25, 1 and 4 mM) were added together with SARS-CoV-2 (0.01 MOI) and incubated for 1h. After 1h, the virus and treatment were rinsed off and the medium was renewed.

Replication: Calu-3 cells were infected with SARS-CoV-2 for 1h (0.01 MOI). After 1h, the virus inoculum was removed and cells were washed to ensure that the viral particles which did not penetrate the cells were rinsed off. Consecutively different concentrations of nCpG-6-PTO were added.

For both set-ups, the detection of viral protein was carried out after 2 days as described above (immunohistochemical detection of a virus-specific antigen using antibodies against SARS-CoV-2S).

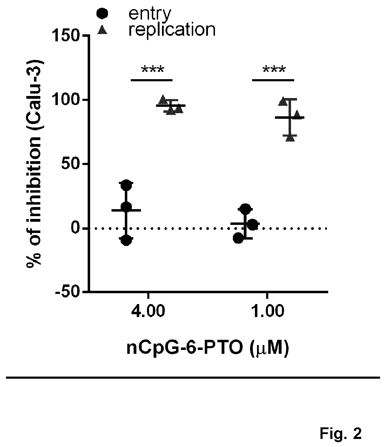

Results are depicted in **Fig. 2**, where it is shown that nCpG-6-PTO prevents replication of SARS-CoV-2 (triangle), but has no impact on virus entry into target cells (circles).

### Example 3:

### The antiviral effect is length-dependent

Comparison of nCpG-6-PTO deletion mutants. In examples 1 and 2 (Figs. 1 and 2) the antiviral efficacy of nCpG-6-PTO was demonstrated. Example 3 shows that there is a correlation between the length of the molecule and the antiviral effect. For this purpose, deletion mutants of nCpG-6-PTO were used.

The procedure was performed as described in example 1. Oligonucleotide molecules of the following different lengths were used as concentrations 0.5, 1, 2, 4µM ODN:
(a) nCpG-6-PTO: 5'- GGG GGG GGG GGG GGG GGG GG -3'
(b) nCpG-6C-PTO: 5'- GGG GGG GGG GGG GG -3'
(c) nCpG-6G-PTO: 5'- GGG GGG -3'

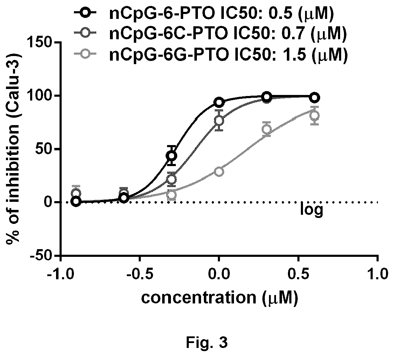

As shown in Fig. 3, the antiviral efficacy clearly depends on the length of the molecule: long molecules displayed stronger effect on virus infection than shorter ones. The IC 50 was the lowest using nCpG-6-PTO (0.5 mM, black circles) and shifted towards lower efficacy with the 6G ODN nCpG-6G-PTO (1.5 mM, light gray circles). Note that concentrations are depicted on a log scale.

### Example 4:

### The antiviral effect depends on the ODN-backbone.

The impact of the ODN backbone was tested by comparing nCpG-6 with a phosphodiester backbone (nCpG-6-PDE, light gray circles) vs. phosphorothioate backbone (nCpG-6-PTO, black circles). Treatment: nCpG-6-PTO or nCpG-6-PDE were applied as concentrations of 0.5, 1, 2, 4µM, respectively. The procedure was performed as described in example 1.

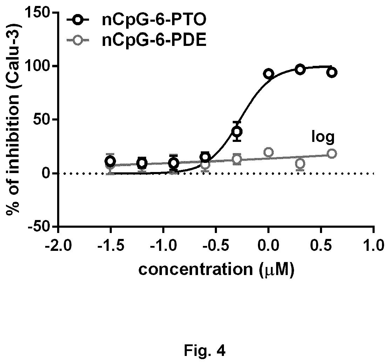

Fig. 4 shows that PTO bonds may convey the antiviral effect.

### Example 5:

### Comparison of virus inhibition of nCpG-6-PTO, AS1411-PTO and AS1411-PDE

Example 5 compares inhibition of replication using the inventive nCpG-6-PTO and two forms of the well-known aptamer AS1411, namely AS1411-PTO and AS1411-PDE. AS1411 (also known as AGRO100) is a G-rich oligonucleotide with phosphodiester bondings (PDE) that has long been established as a potent anti-cancer aptamer. Structurally, AS1411 presumably exists in multiple different G-quadruplex conformations, serving as an example for single oligonucleotides which may adopt multiple G-quadruplex conformations. Treatment was conducted at the following concentrations: 0.5, 1, 2, 4µM of nCpG-6-PTO (black circles), AS1411-PTO (gray circles) and AS1411-PDE (light gray circles), respectively. The procedure was performed as described in example 1.
nCpG-6-PTO: GGG GGG GGG GGG GGG GGG GG
AS1411-PDE (aka AGRO100,): ggt ggt ggt ggt tgt ggt ggt ggt gg
AS1411-PTO: GGT GGT GGT GGT TGT GGT GGT GGT GG

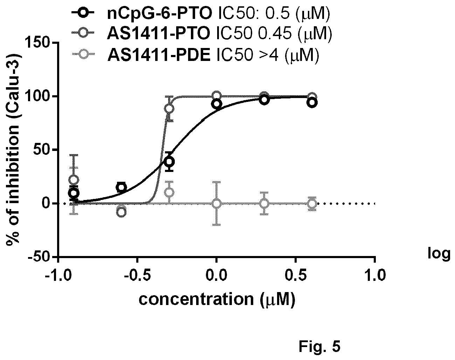

As depicted in **Fig. 5**, nCpG-6-PTO showed high antiviral efficacy. AS1411-PDE, which is being tested as an antiviral drug exhibited no efficacy against SARS-CoV-2. Only when the backbone was replaced by PTO bonds antiviral efficacy became apparent, comparable with nCpG-6-PTO.

### Example 6:

### nCpG-6-PTO also shows anti-viral efficacy against Herpes simplex 1 (HSV-1).

nCpG-6-PTO or nCpG-6-PDE were applied onto human foreskin fibroblasts in conjunction with HSV-1. After two days, the cytopathogenic effect (CPE) of the HSV-1 infection was determined by visual scoring. Treatment was conducted at the following concentrations: 0.5, 1, 2, 4µM.

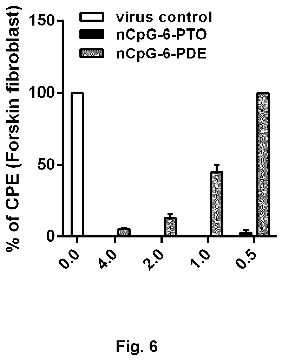

In **Fig. 6** it is shown that nCpG-6-PTO completely suppresses the CPE caused by HSV-1 at the concentration range tested (black bars). Of note, it was found that nCpG-6-PDE also exhibited suppression of CPE of HSV-1 (gray bars), however only at concentrations ≥ 1 µM.

### Example 7:

### nCpG-6-PTO forms quadruplexes (G4)

In order to elucidate a potential mechanism of action, an antibody specific for recognizing DNA and RNA G-quadruplex structures with high selectivity at low nanomolar affinity was used to investigate secondary structure formation of the oligonucleotide molecule of the invention in an in vitro setting.

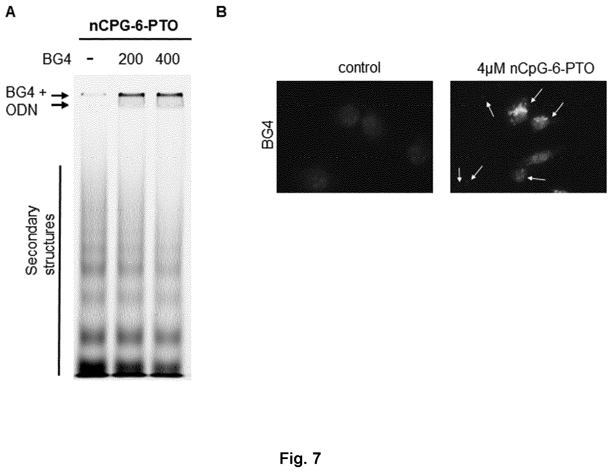

5'-Cy5-labelled nCpG-6-PTO (2µg) was mixed with 200ng or 400ng BG4, an antibody specific to G4 secondary structures (Biozol ABA-AB00174-1.1, Eching, Germany), for 15min at room temperature. After separation by 10% non-denaturing, native PAGE (100 V, corresponding to 14.7 V/cm) with 0.5x TBE fluorescence was captured using the LI-COR Odyssey Gel documentation system (Bad Homburg, Germany). Titration of BG4 with the G4 containing 5'-Cy5-labelled nCpG-6-PTO showed that the antibody binds to the G4-forming DNA (**Fig. 7A**, lanes 2, 3). No complex formation was detected in the absence of BG4 antibody (first lane).

To display G4 structures on the cellular level, A375 cells (human malignant melanoma cell line) were grown on glass coverslips in the presence or absence of 4µM nCpG-6-PTO for 24h. After fixation in 2% paraformaldehyde/PBS, cells were permeabilized and blocked with 0.1% triton-X100/5% normal goat serum in PBS. The primary antibody, BG4 (0.5µg/ml in BSA), was applied to the cells for 90min at room temperature; a mouse IgG1 antibody (Dako) served as control. After incubation with Alexa 488 coupled anti-mouse IgG (Invitrogen) representative images were taken using a Zeiss microscope. All cells examined showed punctate nuclear staining (**Fig. 7B**, right panel, arrows in the experimental condition: 4µM nCpG-6-PTO) not observed in the absence of primary BG4 antibody (**Fig. 7**, left panel, control).

### Example 8:

### nCpG-6-PTO inhibits the SARS-CoV-2 helicase.

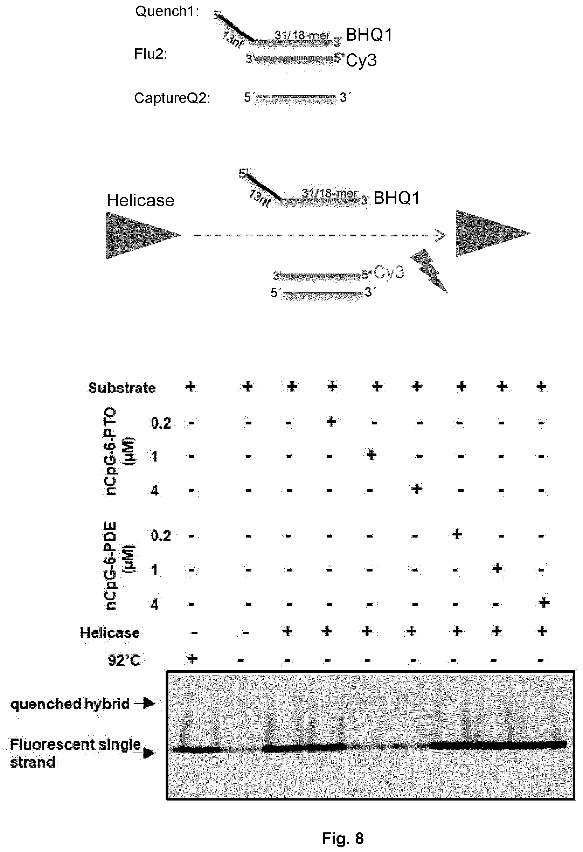

The helicase assay was performed according to the method of Adedeji et al. (Adedeji et al., 2012). In brief, nsp13, the helicase from SARS-CoV-2, was incubated with hybridized primers consisting of Quench2: 5 '-CGCAGTCTTCTCCTGGTGCTCGAACAGTGAC-3'-BHQ1 and Flu2: Cy3-5'-GTCACTGTTCGAGCACCA-3'. Helicase activity separates the hybrids so that the fluorescence of primer Flu2 is no longer quenched. Addition of an excess of CaptureQ2: 5'-GTCACTGTGTGTG as capture probe prevents reannealing of Flu2 with Quench2. Fig. 8, upper panel, depicts a scheme representing the principle of the assay. The helicase assay was conducted using increasing concentrations (0.2 1, 4 mM) of either nCpG-6-PTO or nCpG-6-PDE for comparison. Products were resolved by a 6% non-denaturing-PAGE (polyacrylamide gel electrophoresis), Fig. 8, lower panel. Adding increasing concentrations of nCpG-6-PTO results in inhibition of nsp13, as indicated by increasing amounts of quenched hybrid (see lanes 5, 6 vs. control, lane 2). The addition equal concentrations of nCpG-6-PDE failed to inhibit helicase activity comparably, as indicated by the presence of fluorescent single strands comparable to control.

### Example 9:

### nCpG-6-PTO suppresses IFN type I (IFNβ)-induced STAT-phosphorylation

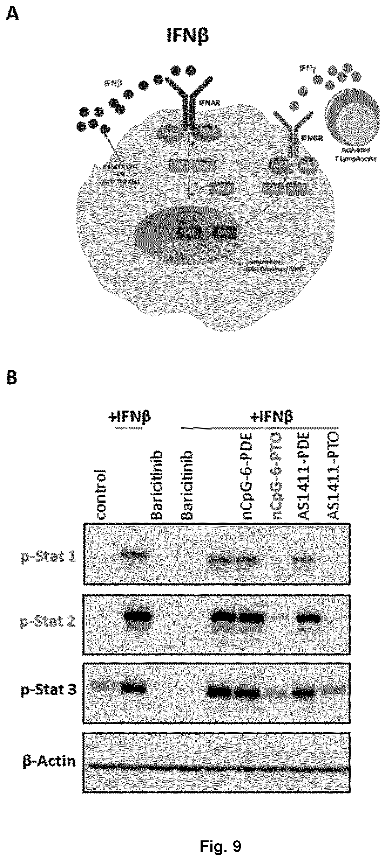

A scheme of the type I interferon signaling pathway is depicted in Fig. 9A (from Gonzalez-Cao *et al.*, 2018). Calu-3 cells were treated with 4µM nCpG-6-PDE, nCpG-6-PTO, AS1411-PTO, AS1411-PDE for 1h and then stimulated with IFNβ (20ng/ml). Baricitinib, a drug for the treatment of rheumatoid arthritis, a well-known inhibitor of janus kinase subtypes JAK1 and JAK2, was used for control (1mM). After 10min, cells were lysed in Strawn Buffer (20mM HEPES [pH7.5], 150mM NaCl, 0.2% Triton X 100, 10% glycerol) supplemented with a protease and phosphatase inhibitors (Roche, Mannheim, Germany) sonicated, boiled for 5min, and then separated on SDS-polyacrylamide gels. Consecutively, proteins were immunoblotted to a PVDF membrane. The membrane was blocked in blocking buffer (TBS [pH7.6], 0.1% Tween-20, 5% nonfat dry milk) for at least 1h at RT followed by incubation with the following primary antibodies: p-Stat-1 (Tyr701), p-Stat-2 (Tyr 690), p-Stat-3 (Tyr705), all from CST (Frankfurt, Germany) and anti-bactin (Santa Cruz, Biotechnology, Heidelberg, Germany) as control for equal loading. Bound primary antibodies were detected by using rabbit anti-goat IgG-horseradish peroxidase conjugates (Dako, Frankfurt, Germany) and visualized with the LumiGlo detection system (CST). Results as shown in **Fig. 9B** clearly demonstrate an inhibition of Stat 1 and Stat2 phosphorylation by nCpG-6-PTO but not by nCpG-6-PDE; likewise, the PTO form of G-rich, quadruplex-forming AS1411 sufficiently suppressed tyrosine phosphorylation of both Stat 1 and Stat2, while the PDE form failed to do so.

### Example 10:

### nCpG-6-PTO suppresses IFN type II (IFNg)-induced JAK2 and STAT-phosphorylation

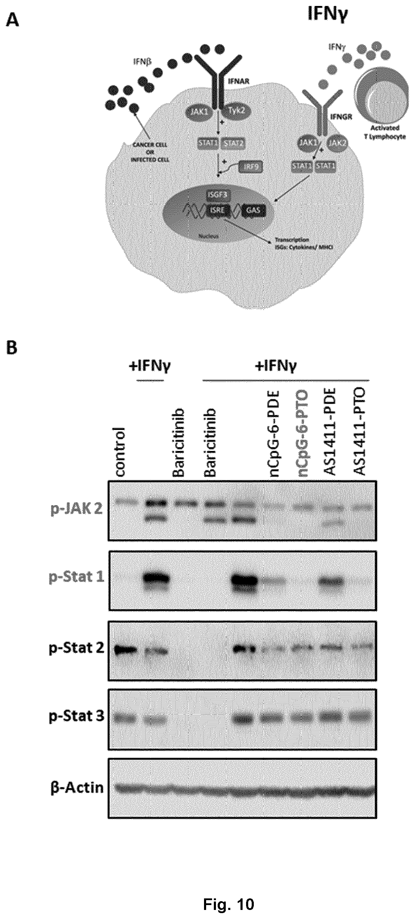

A scheme of the type II interferon signaling pathway is depicted in **Fig. 10A** (from Gonzalez-Cao *et a*/*.*, 2018). For experimental setup, see above, example 9: Calu-3 cells were treated with 4µM nCpG-6-PDE, nCpG-6-PTO, AS1411-PTO, AS1411-PDE for 1h and then stimulated with IFNg (20ng/ml). After resolving on SDS page, phosphorylation was detected using p-Stat-1 (Tyr701), p-Stat-2 (Tyr 690), p-Stat-3 (Tyr705) and p-JAK2 (Tyr1007/Tyr1008) antibodies, all from CST (Frankfurt, Germany), and anti-bactin (Santa Cruz, Biotechnology, Heidelberg, Germany) as control for equal loading.

Western blot analysis, **Fig. 10B****,** shows strong downregulation of tyrosine phosphorylation of canonical signaling molecules p-JAK2 and pStat1. Also, suppressive effects on pStat2/3 were found, confirming the partial overlap of type I and type II signaling pathways (Garcia-Diaz *et al.*, 2017).

### Example 11:

### nCpG-6-PTO suppresses Interleukin 6 (IL-6)-mediated STAT-3 phosphorylation

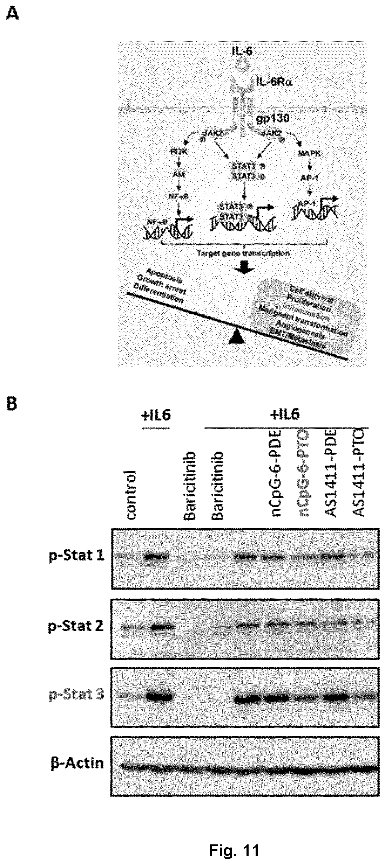

A scheme of the interleukin 6 (IL-6) signaling pathway is depicted in **Fig. 11A** (from Jin *et al.*, 2017). For experimental setup, see above, example 9: Calu-3 cells were treated with 4µM nCpG-6-PDE, nCpG-6-PTO, AS1411-PTO, AS1411-PDE for 1h and then stimulated with IL-6 (20ng/ml). After resolving on SDS page, phosphorylation was detected using p-Stat-1 (Tyr701), p-Stat-2 (Tyr 690), p-Stat-3 (Tyr705) antibodies, all from CST (Frankfurt, Germany), and anti-bactin (Santa Cruz, Biotechnology, Heidelberg, Germany) as control for equal loading.

Western blot analysis, **Fig. 11B**, shows strong downregulation of tyrosine phosphorylation Western blot analysis shows downregulation of pStat3. Also, suppressive effects on pStat1 were found.

## Claims

1. An oligonucleotide molecule having from 10 to 50 nucleotides comprising at least one G-quartet forming motif comprising 10 to 20 nucleotide residues, wherein at least 60% of said G-quartet forming motive forming residues are guanosine or deoxyguanosine residues, and wherein the molecule inhibits viral or bacterial replication and/or exerts anti-inflammatory effects in mammalian cells.

2. The oligonucleotide molecule according to claim 1, wherein > 70% of the residues are guanosine or deoxyguanosine residues.

3. The oligonucleotide molecule according to claim 1 or 2, wherein the oligonucleotide is synthesized from deoxynucleotides.

4. The oligonucleotide molecule according to any one of the preceding claims, wherein at least one of the guanosine or deoxyguanosine residues is chemically modified.

5. The oligonucleotide molecule according to claim 4, wherein the chemical modification is a phosphate backbone modification.

6. The oligonucleotide molecule according to claim 5, wherein thiophosphoryl substitutions are selected from phosphorothioate or phosphorodithioate, and wherein thiophosphoryl substitutions replace at least 35% of the phosphodiester linkages in the sugar-phosphate backbone of the oligonucleotide molecule.

7. The oligonucleotide molecule according to any of the preceding claims, wherein the oligonucleotide comprises at least four consecutive triplets of guanosine or deoxyguanosine residues.

8. The oligonucleotide molecule according to any of the preceding claims, wherein the molecule comprises any one of the sequences set forth in SEQ ID NOs. 1 to 4.

9. Pharmaceutical composition comprising at least one oligonucleotide molecule of any of the preceding claims combined with at least one of a pharmaceutically acceptable excipient, carrier, adjuvant or combination thereof.

10. Kit comprising at least one oligonucleotide molecule according to any one of the claims 1 to 8.

11. Oligonucleotide molecule according to anyone of one of claims 1 to 8, pharmaceutical composition according to claim 9 or kit according to claim 10 for use in treatment of a disease caused by a viral or a bacterial infection and/or of inflammation associated therewith.

12. The use according to claim 11, wherein the oligonucleotide molecule induces inhibition of viral replication in a mammalian cell.

13. The use according to claim 12, wherein the viral infection is caused by a virus selected from the group comprising HS-1 virus, HCN virus, Adenovirus, Zika virus, hepatitis C virus, West Nile virus, influenza virus, RSV virus, Paramyxo-Virus, HIV virus, coronaviruses, such as SARS-CoV-1, SARS-CoV-2 and MERS-CoV.

14. The use according to claim 12, wherein the disease is a viral infection of the respiratory tract.

15. The use according to claim 11, wherein the treatment of the viral or bacterial disease-associated inflammation results from interference with the type I interferon (IFN) and/or the type II IFN pathway or from suppression with interleukin mediated signaling.

16. The use of claim 11, wherein in the oligonucleotide molecule at least one of the guanosine or deoxyguanosine residues is chemically modified, wherein the chemical modification is a phosphate backbone modification, and wherein the oligonucleotide comprises at least four consecutive triplets of guanosine or deoxyguanosine residues.

17. The use of claim 11, wherein the molecule is administered in a pharmaceutically acceptable route selected from the group consisting of orally, parenterally, enterally, via the ophthalmic or nasal route, or topically and combinations thereof. (Other routes of application, e.g. topical as cream or lotion (for treatment of HSV infections), as spray, as shampoo, as eye drops, as suppositories, transdermal patch, nail polish should be also considered.
